# EUROPEAN PATENT APPLICATION

(11) **EP 3 875 435 A1**
(43) Date of publication of application: **08.09.2021**
(21) Application number: 19878775.6
(22) Date of filing: 29.10.2019
(51) Int. Cl.: C02F 1/461, A61L 2/18

(54) **HYDROGEN WATER AND STERILE WATER GENERATION DEVICE**

(30) Priority: 30.10.2018 WO PCT/JP2018/040218
(71) Applicant: Gohda Water Treatment Technology Co., Inc., Tokyo 104-0041 (JP); Lee, Jae Yong, Goyang-si, Gyeonggi-do 10424 (KR)
(72) Inventor: GOHDA, Toshihisa, Tokyo 104-0041 (JP); LEE, Jae Yong, Goyang-si, Gyeonggi-do, 10424 (KR)
(74) Representative: Brand Murray Fuller LLP
(86) International application number: PCT/JP2019/042316
(87) International publication number: WO 2020/090795

(57) **Abstract**

The present invention relates to a hydrogen water and sterile water-generating device that generates hydrogen water and sterile water by electrolysis. The hydrogen water and sterile water-generating device includes an electrolysis part that has at least two electrodes and electrolyzes water, a water introducing channel that introduces water into the electrolysis part, and a switch mechanism that switches the polarity of the electrodes between positive and negative, and hydrogen water and sterile water are generated in the same path by the switch mechanism switching the polarity of the electrodes between positive and negative during electrolysis.

## Description

### Technical Field

The present invention relates to a hydrogen water and sterile water-generating device that generates hydrogen water and sterile water by electrolysis, a method of generating mixed water of hydrogen water and sterile water, and a set of the generation device and a baby bottle.

### Background Art

Recently, it has been shown that hydrogen in hydrogen water serves to remove active oxygen in the human body, and attempts have been made to generate and use water containing more dissolved hydrogen. As a result, there is an increasing demand for hydrogen water, and methods and devices are required which allow hydrogen water to be easily used (Patent Document 1). Meanwhile, with the recent raised awareness of hygiene, there is also an increasing demand for sterilization of foods or beverages, containers for foods or beverages, or the like. The demand for sterilization includes not only the demand for sterilization in the manufacturing process but also the recently increasing demand for personal or home sterilization of foods or beverages people take regularly, containers or cooking utensils used regularly, or the like. Examples of items to be sterilized include a food such as a vegetable, a reusable container such as a plastic bottle, a baby bottle or a water bottle, a dish, a cooking utensil such as a cutting board. There are various other items to be sterilized, such as a portable hydrogen water-generating device or a boiling pot. However, there has not been provided a method or device that allows sterilization to be easily performed personally or at home.

As for the baby bottle that needs to be sterilized at home, for example, any remaining baby formula is susceptible to bacteria growth. Therefore, in order to prevent a baby from taking in bacteria, the baby bottle is now sterilized in three ways, the boiling disinfection, the chemical disinfection, and the microwave disinfection. The boiling disinfection is a process in which water is boiled in a pot that is sufficiently deep for the baby bottle to be immersed in the water, and the baby bottle is immersed in the boiling water for a certain time. The chemical disinfection is a process in which a chemical solution is prepared by putting a predetermined amount of water and a chemical solution in a container for chemical solution preparation, and the baby bottle is immersed in the prepared chemical solution for a certain time. The microwave disinfection is a process in which a small amount of water and the baby bottle are put in a dedicated container for microwave disinfection and microwaved for a certain time (Patent Document 2). However, any of these processes requires a large dedicated container. In addition, the boiling disinfection requires a stove for boiling water, and the microwave disinfection requires a microwave oven. Therefore, these disinfection processes are not easy to perform. In particular, it is not easy to carry a large container when going out, and it is not easy to use a stove or microwave oven at a location away from home, so that it is difficult to disinfect a baby bottle at a location away from home. In addition, a parent going out with a baby cannot use a baby bottle once used again and therefore is required to carry a plurality of baby bottles.

This problem is not peculiar to the baby bottle. For example, a beverage may be contained and carried in a plastic bottle, and the plastic bottle may be reused after the beverage is consumed. In such a case, bacteria are likely to grow particularly in summer, and the plastic bottle is desirably sterilized depending on the season or content. However, there has not been proposed a method for easily sterilizing a container such as a plastic bottle in such a situation. Furthermore, there has not been proposed a method of easily sterilizing a vegetable to be cooked at home or a method of easily sterilizing a dish or a cooking utensil such as a cutting board at home. Furthermore, as for the electric pot or insulation pot, if the hot water is left standing, bacteria may grow while the hot water is kept warm or during an extended use, for example. However, there has not been proposed a method of easily sterilizing such a pot. In addition, although a portable hydrogen water-generating device is used as a hydrogen water-generating device, such a device has only the function of generating hydrogen water, and there has not been proposed a device that generates sterile water.

### Prior Art Documents

### Patent Documents

Patent Document 1: Japanese unexamined Patent Application Publication No. 10-296262
Patent Document 2: Japanese unexamined Patent Application Publication No. 2007-061453

### Summary of the Invention

### Object to be Solved by the Invention

The present invention is intended to solve the problems described above, and an object of the present invention is to provide a device that can readily generate hydrogen water and sterile water having a sterilization effect and can be easily operated. Another object of the present invention is to make the above-described device portable.

### Means to Solve the Object

The present inventors have conducted research and development about hydrogen water, such as hydrogen water-generating devices and hydrogen water utilization methods, for a long time. In the research and development, the present inventors have focused attention to the sterilization effect of the hydroxide ion generated when hydrogen water is generated. There have already been methods of generating a highly alkaline electrolyzed water and a highly acid electrolyzed water by electrolyzing water containing an additive salt. However, hydrogen water is not generated in these methods. Although combining hydrogen water and sterile water has not been contemplated, the present inventors have thought that, if a device is developed which can easily generate not only hydrogen water but also sterile water that is rich in hydroxide ion, and the hydrogen water and the sterile water are used in combination, the application area of hydrogen water will be expanded, and sterile water containing hydroxide ions will be more popularly used. And the present inventors have studied the idea. Then, the present inventors have found that, if the polarities of the electrodes used for electrolysis are inverted to switch between the generation of hydrogen water and the generation of sterile water, and the hydrogen water and the sterile water are generated in the same path, a device that is simple in structure and easy to operate that can easily generate hydrogen water and sterile water as required can be implemented. In addition, the present inventors have found that this device not only can separately generate hydrogen water and sterile water but also can generate mixed water of hydrogen water and sterile water. The present invention has been made in this way.

Specifically, the present invention is defined by the following items.
(1) A hydrogen water and sterile water-generating device, comprising an electrolysis part that has two electrodes and electrolyzes water, a water introducing channel that introduces water into the electrolysis part, a switch mechanism that switches a polarity of the electrodes between positive and negative, and an ion exchange membrane arranged between the two electrodes, wherein the electrodes are electrodes having a through-hole, the water introducing channel is connected to only one of the electrodes from outside of the electrolysis part, and wherein hydrogen water and sterile water are generated in a same path by a switching of the switch mechanism that switches a polarity of the electrodes between positive and negative during electrolysis.
(2) The hydrogen water and sterile water-generating device according to "1", wherein the hydrogen water and sterile water-generating device comprises a container for containing water, and has a structure wherein the electrolysis part is in communication with an inside of the container, so that the water in the container is introduced into the electrolysis part, and the water in the container is changed to hydrogen water, sterile water, or mixed water of hydrogen water and sterile water by electrolysis of the introduced water.
(3) The hydrogen water and sterile water-generating device according to "1", wherein the hydrogen water and sterile water-generating device comprises an opening part to which a mouth part of the container for containing water is removably attached, and has a structure wherein the electrolysis part is in communication with the opening part, so that the water in the container is introduced into the electrolysis part when the container is attached to the opening part, and the water in the container is changed to hydrogen water, sterile water, or mixed water of hydrogen water and sterile water by electrolysis of the introduced water.
(4) The hydrogen water and sterile water-generating device according to any one of "1" to "3", wherein the ion exchange membrane arranged between the two electrodes are arranged between the two electrodes in contact with the two electrodes.
(5) The hydrogen water and sterile water-generating device according to any one of "1" to "4", wherein a width of the through-hole of the electrodes is 0.1 to 3 mm.
(6) A method of generating mixed water of hydrogen water and sterile water, comprising introducing water into a water electrolysis part from only a side of one of the electrodes, the electrolysis part comprising two electrodes and an ion exchange membrane arranged between the two electrodes, alternately generating hydrogen water and sterile water in a same path by switching a polarity of the electrodes between positive and negative, and mixing the hydrogen water and the sterile water to generate mixed water of hydrogen water and sterile water.
(7) The method of generating mixed water of hydrogen water and sterile water according to "6", comprising using the hydrogen water and sterile water-generating device according to any one of "1" to "5".
(8) The hydrogen water and sterile water-generating device according to "2", wherein the hydrogen water and sterile water-generating device is an electric pot comprising a water boiling mechanism.
(9) The hydrogen water and sterile water-generating device according to "3", wherein the container is a baby bottle.
(10) A kit of a baby bottle and a hydrogen water and sterile water-generating device, comprising a baby bottle and the hydrogen water and sterile water generation device according to "9".

The present invention is also defined by the following items.
(1) A hydrogen water and sterile water-generating device, comprising an electrolysis part that has at least two electrodes and electrolyzes water, a water introducing channel that introduces water into the electrolysis part, and a switch mechanism that switches a polarity of the electrodes between positive and negative, wherein hydrogen water and sterile water are generated in a same path by a switching of the switch mechanism that switches a polarity of the electrodes between positive and negative during electrolysis.
(2) The hydrogen water and sterile water-generating device according to "1", wherein the hydrogen water and sterile water-generating device comprises a container for containing water, and has a structure wherein the electrolysis part is in communication with an inside of the container, and the water in the container is introduced into the electrolysis part, so that the water in the container is changed to hydrogen water, sterile water, or mixed water of hydrogen water and sterile water by electrolysis of the introduced water.
(3) The hydrogen water and sterile water-generating device according to "1", wherein the hydrogen water and sterile water-generating device comprises an opening part to which a mouth part of the container for containing water is removably attached, and has a structure wherein the electrolysis part is in communication with the opening part, so that the water in the container is introduced into the electrolysis part when the container is attached to the opening part, and the water in the container is changed to hydrogen water, sterile water, or mixed water of hydrogen water and sterile water by electrolysis of the introduced water.
(4) The hydrogen water and sterile water-generating device according to any one of "1" to "3", wherein the electrolysis part has two electrodes, the water introducing channel that introduces water into the electrolysis part is connected to only one of the electrodes from outside of the electrolysis part, and the electrode to which the water introducing channel is connected is an electrode having a through-hole.
(5) A method of generating mixed water of hydrogen water and sterile water, comprising introducing water into a water electrolysis part, the electrolysis part comprising at least two electrodes, alternately generating hydrogen water and sterile water in a same path by switching a polarity of the electrodes between positive and negative, and mixing the hydrogen water and the sterile water to generate mixed water of hydrogen water and sterile water.
(6) The method of generating mixed water of hydrogen water and sterile water according to "5", comprising using the hydrogen water and sterile water-generating device according to any one of "1" to "4".
(7) The hydrogen water and sterile water-generating device according to "2", wherein the hydrogen water and sterile water-generating device is an electric pot comprising a water boiling mechanism.
(8) The hydrogen water and sterile water-generating device according to "3", wherein the container is a baby bottle.
(9) A kit of a baby bottle and a hydrogen water and sterile water-generating device, comprising a baby bottle and the hydrogen water and sterile water-generating device according to "8".

### Effect of the Invention

The hydrogen water and sterile water-generating device according to the present invention can generate hydrogen water and sterile water by electrolysis by itself, and can generate the hydrogen water and the sterile water in the same path. Therefore, the hydrogen water and sterile water-generating device is simple in structure, can produce hydrogen water and sterile water in a simple operation, and can easily mix the hydrogen water and the sterile water. In addition, the hydrogen water and sterile water-generating device can be provided with a container having a pot-like or other shape, and water put in the container can be turned into hydrogen water, sterile water, or mixed water of hydrogen water and sterile water. If the container is configured to be removable, water in a baby bottle or a reusable container such as a plastic bottle can be turned into hydrogen water, sterile water, or mixed water of hydrogen water and sterile water, for example. Therefore, after the hydrogen water is consumed, the inside of the container can be sterilized. Furthermore, the hydrogen water and sterile water-generating device can be reduced in size so that the device is portable, so that the device can be used at a location away from home. Furthermore, with the generation device and the generation method according to the present invention, hydrogen water and sterile water can be alternately generated, so that a pipe for transporting the hydrogen water generated by the generation device according to the present invention or a storage tank can also be sterilized to prevent generation of grime. Furthermore, with the generation device and the generation method according to the present invention, hydrogen water and sterile water can be generated in the same path, so that mixed water of hydrogen water and sterile water can be easily generated. When the ratio of hydrogen water is high, the mixed water produced in this way can prevent generation of grime during an extended use of a transportation pipe or a storage tank while having an effect of hydrogen water. Even when the ratio of sterile water is high, the mixed water can remove or clean off a fine particle since the mixed water contains hydrogen water.

### Brief Description of Drawings

[Figure 1] Figure 1(a) is a schematic diagram (cross-sectional view) showing a hydrogen water and sterile water-generating device according to an embodiment of the present invention. Figure 1(b) is a top view showing an example of an electrode of the hydrogen water and sterile water-generating device according to the present invention.
[Figure 2] Figure 2(a) is a schematic diagram (cross-sectional view) showing a hydrogen water and sterile water-generating device according to the embodiment of the present invention that includes a container. Figure 2(b) is a schematic diagram (cross-sectional view) showing the hydrogen water and sterile water-generating device in Figure 2(a) that additionally includes a heating mechanism.
[Figure 3] Figure 3 is a schematic diagram (cross-sectional view) showing the hydrogen water and sterile water-generating device according to the embodiment of the present invention that includes an opening part to which a container can be removably attached.
[Figure 4] Figures 4(a) to 4(c) are schematic diagrams (cross-sectional view) showing a way in which the hydrogen water and sterile water-generating device in Figure 3 is attached to a container.
[Figure 5] Figure 5(a) and 5(b) are schematic diagrams (cross-sectional view) showing a way in which a nipple is sterilized in a case where the container is a baby bottle.
[Figure 6] Figure 6(a) is a schematic diagram (cross-sectional view) showing the hydrogen water and sterile water-generating device according to the present invention that is covered with a lid. Figure 6(b) is a schematic diagram (cross-sectional view) showing a nipple placed on the lid.

### Mode of Carrying Out the Invention

A hydrogen water and sterile water-generating device according to the present invention is characterized in that the hydrogen water and sterile water-generating device is a device comprising an electrolysis part that has at least two electrodes and electrolyzes water, a water introducing channel that introduces water into the electrolysis part, and a switch mechanism that switches a polarity of the electrodes between positive and negative, and hydrogen water and sterile water are generated in a same path by the switch mechanism switching a polarity of the electrodes between positive and negative during electrolysis. The expression "generated in a same path" means that the path in which raw material water (water yet to be electrolyzed) flows into the electrolysis part through the introducing channel for introducing water into the electrolysis part and is electrolyzed to generate hydrogen water, and the generated hydrogen water is discharged from the electrolysis part is the same as the path in which raw material water (water yet to be electrolyzed) flows into the electrolysis part through the introducing channel for introducing water into the electrolysis part and is electrolyzed to generate sterile water, and the generated sterile water is discharged from the electrolysis part. Furthermore, a hydrogen water and sterile water-generating device according to the present invention is characterized in that the hydrogen water and sterile water-generating device is a device comprising an electrolysis part that has two electrodes and electrolyzes water, a water introducing channel that introduces water into the electrolysis part, a switch mechanism that switches a polarity of the electrodes between positive and negative, and an ion exchange membrane arranged between the two electrodes, wherein the electrodes are electrodes having a through-hole, the water introducing channel is connected to only one of the electrodes from outside of the electrolysis part, and hydrogen water and sterile water are generated in a same path by the switch mechanism switching a polarity of the electrodes between positive and negative during electrolysis. The water introduced into the electrolysis part is not limited to water at room temperature but can be so-called cold water or hot water. In other words, the water is water that has a temperature of 0 to 100°C and is in the liquid state, including typical potable water, such as tap water, natural water, distilled water, ion-exchanged water or water processed with a reverse osmotic membrane. Generally, when electrolyzing water, different kinds of electrolyzed water are generated from each side of the two electrodes. However, the generating device according to the present invention includes a switch mechanism that switches the polarities of the electrodes between positive and negative, and different kinds of water, hydrogen water and sterile water, can be generated in the same path by the switch mechanism switching the polarities of the electrodes between positive and negative. The electrodes of the hydrogen water and sterile water-generating device according to the present invention are not particularly limited, and may be a titanium plate, a platinum plate, or a titanium plate plated with platinum.

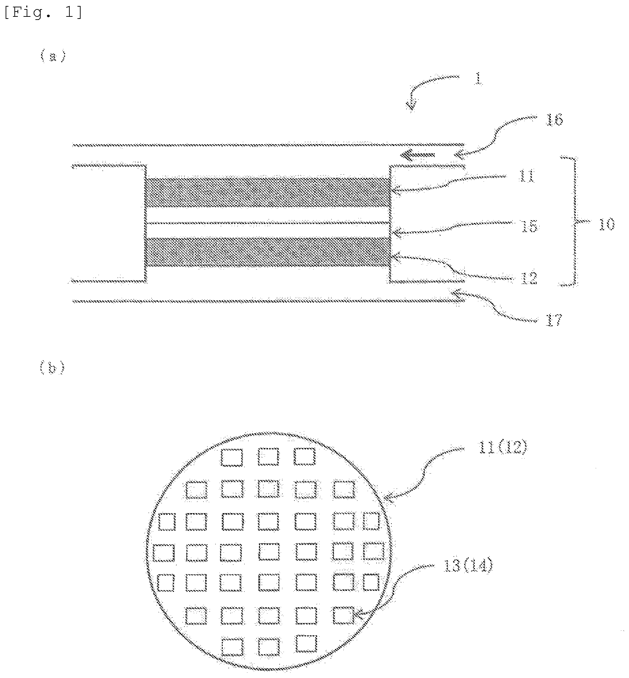

A hydrogen water and sterile water-generating device according to the present invention will be described with reference to the drawings. Figure 1(a) is a schematic diagram showing a hydrogen water and sterile water-generating device according to an embodiment of the present invention. An electrolysis part 10 of a hydrogen water and sterile water-generating device 1 has an electrode (A) 11, an electrode (B) 12, and an ion exchange membrane 15. The electrode (A) 11 and the electrode (B) 12 are each an electrode plate having a large number of through-holes as shown in Figure 1(b). The ion exchange membrane 15 is a cation exchange membrane. Although the ion exchange membrane 15 is in contact with the electrode (B) 12, and there is a gap between the ion exchange membrane 15 and the electrode (A) 11 in Figure 1(a), the ion exchange membrane 15 may be arranged between the electrode (A) 11 and the electrode (B) 12 in contact with both the electrodes. Water having flowed through a water introducing channel 16 for introducing water into the electrolysis part 10 flows into the electrolysis part through through-holes 13 of the electrode (A) 11 and is electrolyzed. In this way, with the hydrogen water and sterile water-generating device 1, the electrolysis part 10 consists of two electrodes (the electrode (A) 11 and the electrode (B) 12) and an ion exchange membrane arranged between the two electrodes, and the water introducing channel 16 for introducing water into the electrolysis part 10 is connected to only one of the electrodes (only the electrode (A) 11) from outside of the electrolysis part 10, and no water introducing channel is provided for the other electrode (the electrode (B) 12). The electrode (A) 11, to which the water introducing channel 16 is connected, is an electrode having through-holes. The electrolysis part 10 is configured so that no water leaks to the side surface through the electrode (A) 11, the electrode (B) 12 and the gaps between the ion exchange membrane 15 and the electrodes, and the ion exchange membrane 15 is arranged to prevent water introduced into the through-holes of the one electrode (A) 11 from directly reaching the other electrode (B) 12. For example, when a wall part that surrounds the electrode (A) 11, the ion exchange membrane 15 and the electrode (B) 12 is provided, and the ion exchange membrane 15 is arranged between the two electrodes in contact therewith, the ion exchange membrane is shaped or sized to block the through-holes of the one electrode (A) 11, for example. The hydrogen water and sterile water-generating device according to the present invention has only to have at least two electrodes and an ion exchange membrane arranged between the two electrodes, as the hydrogen water and sterile water-generating device 1. The hydrogen water and sterile water-generating device according to the present invention can have another electrode or a plurality of sets of two electrodes and an ion exchange membrane arranged therebetween, as far as the hydrogen water and sterile water-generating device has the effects and advantages of the present invention. Provided that the electrode (A) 11 is a negative electrode (minus electrode), and the electrode (B) 12 is a positive electrode (plus electrode), hydrogen ions (H⁺) generated by electrolysis of water are attracted by the electrode (A) 11 and turned into hydrogen molecules, which are dissolved in the water in the introducing channel 16 through the through-holes 13. In this way, the hydrogen water and sterile water-generating device 1 generates hydrogen water. Since there is the ion exchange membrane 15 between the electrode (A) 11 and the electrode (B) 12, the water flowing into the electrolysis part 10 does not leak to under the electrode (B) 12, and ozone gas (O₃) is discharged to a gas discharge channel 17 through through-holes 14 of the electrode (B) 12. The gas discharge channel 17 is in communication with the through-holes of the electrode (B) 12, to which the water introducing channel is not connected, at one end thereof, and therefore, the gas generated at the electrode (B) 12 can be discharged to a gas destination. The electrode having a large number of through-holes is not particularly limited, and may be a metal plate having a large number of punched holes, a mesh-like metal plate such as an expanded metal, a lattice metal plate, a metal plate having longitudinal or lateral slits, or a metal plate formed by a metal fiber, for example. While the size of the electrode plate is not particularly limited, the thickness of the electrode plate may fall within a range from 0.3 to 3 mm, a range from 0.3 to 2.5 mm, or a range from 0.4 to 2.3 mm, for example. The type or size of the ion exchange membrane is not particularly limited. For example, an ion exchange membrane having a based material made of styrene or the like into which an ion exchange group such as a sulfonic group or a quaternary ammonium group is introduced, or a fluorine-based ion exchange membrane such as Nafion (registered trademark) may be used, and the ion exchange membrane may be a cation exchange membrane or an anion exchange membrane. The thickness of the ion exchange membrane may fall within a range from 0.1 to 0.7 mm, a range from 0.1 to 0.5 mm, or a range from 0.1 to 0.3 mm, for example.

The hydrogen water and sterile water-generating device 1 according to the present invention includes a switch mechanism (not shown) that switches the polarity of an electrode between positive and negative. As the switch mechanism, any conventionally known mechanism, such as an electrode reversal circuit, can be used as required. In the hydrogen water and sterile water-generating device 1, the switch mechanism switches the polarities of the electrode (A) 11 and the electrode (B) 12 between positive and negative to set the electrode (A) 11 to be a positive electrode (plus electrode) and the electrode (B) 12 to be a negative electrode (minus electrode). In this case, water having flowed through the water introducing channel 16 for introducing water into the electrolysis part 10 flows into the electrolysis part through the through-holes 13 of the electrode (A) 11 and is electrolyzed, and a hydroxide ion (OH⁻) generated by the electrolysis is attracted by the electrode (A) 11 and moves into the water in the introducing channel 16 through the through-hole 13. In this way, sterile water containing many hydroxide ions (OH⁻) is generated. With the hydrogen water and sterile water-generating device 1, both the hydrogen water and the sterile water are generated in the same path including the introducing channel 16, the electrolysis part 10 and then the introducing channel 16 again. When the hydrogen water generated by the hydrogen water and sterile water-generating device 1 is stored in a storage tank through a pipe for hydrogen water transportation, the pipe and the storage tank can be sterilized by switching from generation of hydrogen water to generation of sterile water after a certain period of time and flowing sterile water through the transportation pipe and the storage tank, so that generation of grime can be prevented. Therefore, if the device 1 according to the present invention is used as a floor-mounted hydrogen water-generating device having a storage tank, the necessity or frequency of cleaning of the storage tank or the pipe can be reduced. In addition, mixed water of hydrogen water and sterile water can be generated in the storage tank by switching between the generation of hydrogen water and the generation of sterile water at regular time intervals. In the generation of the mixed water, for example, the ratio between the duration of the generation of hydrogen water and the duration of the generation of sterile water, or the ratio between the amount of water processed to generate hydrogen water and the amount of water processed to generate sterile water, can be 97:3 to 3:97. When a higher priority is given to the effect of the hydrogen water in the mixed water, the ratio can be preferably 97:3 to 70:30. When a higher priority is given to the effect of the sterile water, the ratio can be 30:70 to 3:97. With the hydrogen water and sterile water-generating device according to the present invention, the water introducing channel is provided so as to supply water only to one of the two electrodes, and only a gas resulting from gasification is discharged from the other electrode, so that a desired electrolyzed water (hydrogen water or sterile water) can be solely obtained. Therefore, there is no need to supply unwanted water in order to obtain a desired electrolyzed water, and an electrolyzed water different from the desired electrolyzed water is not discharged. Therefore, there is no need for a structure or operation for supply or discharge of excess water, and the structure and operation of the device is simplified. In addition, there is no need for an environment ready for the supply or discharge of excess water, so that the use environment is not limited. This characteristic is particularly preferable for a portable device, a home-use device or the like that is used in an environment (such as a location away from home or a room away from the kitchen and bathroom or the like) where it is not easy to supply water or process discharged water. According to the present invention, the width of the through-hole of the electrode is preferably small, and is preferably 0.1 to 3 mm, or more preferably 0.1 to 2 mm. The porosity of the electrode is preferably 35 to 75%, or more preferably 40 to 70%. In general, when electrolysis is achieved with two electrodes separated by an ion exchange membrane, supplying water to both the electrodes increases the efficiency of the electrolysis and promotes the ionization. According to the present invention, however, hydrogen water or sterile water can be efficiently generated by supplying water to only one of the electrodes. Here, the "width of the through-hole" refers to the minimum distance between two parallel straight lines placed on the opposite sides of the figure of the opening part of the through-hole to be tangent to the figure. The shape of the opening part is not particularly limited and can be a circle, an ellipse, a polygon such as a triangle or a rectangle, an irregular shape, or a slit-like shape, for example. The length of the through-hole of the electrode can be 0.1 to 7 mm or 0.1 to 6 mm, for example. Here, the "length of the through-hole" refers to the maximum distance between two parallel straight lines placed on the opposite sides of the figure of the opening part of the through-hole to be tangent to the figure. The "porosity" refers to the ratio of the total surface area of the holes to the overall surface area of the electrode (the total surface area of the holes/the overall surface area of the electrode). The electrodes configured as described above allow efficient dissolution of hydrogen in water while preventing occurrence or growth of bubbles and allow efficient generation of a hydroxide ion when generating sterile water.

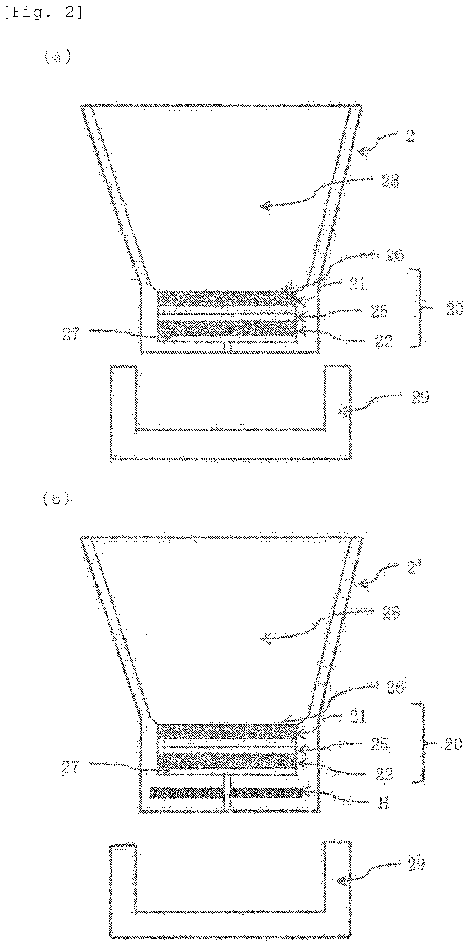

A hydrogen water and sterile water-generating device according to another embodiment of the present invention is characterized in that the hydrogen water and sterile water-generating device comprises a container for containing water, an electrolysis part is in communication with the inside of the container, the water in the container is introduced into the electrolysis part, and the water in the container is turned into hydrogen water, sterile water, or mixed water of hydrogen water and sterile water by electrolysis of the introduced water. Figure 2(a) is a schematic diagram showing an example of the hydrogen water and sterile water-generating device according to this embodiment of the present invention. An electrolysis part 20 of a hydrogen water and sterile water-generating device 2 has an electrode (A) 21, an electrode (B) 22, and an ion exchange membrane 25. The hydrogen water and sterile water-generating device 2 is the same as the hydrogen water and sterile water-generating device 1 shown in Figure 1(a) in the fact that the electrode (A) 21 and the electrode (B) 22 are each a porous electrode plate and the structure of the electrodes, that the ion exchange membrane 25 is a cation exchange membrane, the relationship between the electrode (A) 21 and the electrode (B) 22, the ion exchange membrane 25 and the water introducing channel, that any conventionally known mechanism can be used as the switch mechanism as required, and that an anion exchange membrane can also be used. The hydrogen water and sterile water-generating device 2 includes a container 28, and the electrolysis part 20 is in communication with the container 28. Once water is put in the container 28, some of the water is introduced into the electrolysis part 20. In this embodiment, an introducing channel 26 is a part of the container 28. When water is put in the container 28, and a voltage is applied between the electrode (A) 21 as a negative electrode (minus electrode) and the electrode (B) 22 as a positive electrode (plus electrode), the water introduced into the electrolysis part 20 through the through-holes of the electrode (A) 21 is electrolyzed. Generated hydrogen ions (H⁺) are attracted by the electrode (A) 21 and turned into hydrogen molecules, which are dissolved in the water in the container 28 through the through-holes of the electrode (A) 21. In this way, by continuing the electrolysis for a certain time, the water in the container 28 can be turned into hydrogen water. Ozone gas (O₃) is discharged to a gas discharge channel 27 through the through-holes of the electrode (B) 22. The gas discharge channel 27 is in communication with the through-holes of the electrode (B) 22, to which the water introducing channel is not connected, at one end thereof and is in communication with the outside of the hydrogen water and sterile water-generating device 2 at the other end thereof. Therefore, the gas generated at the electrode (B) 22 can be discharged to the outside of the hydrogen water and sterile water-generating device 2. The container 28 may have a lid.

When water is put in the container 28, and the polarities of the electrode (A) 21 and the electrode (B) 22 are switched between positive and negative by the switch mechanism to set the electrode (A) 21 to be a positive electrode (plus electrode) and set the electrode (B) 22 to be a negative electrode (minus electrode), hydroxide ions (OH⁻) generated by the electrolysis are attracted by the electrode (A) 21 and spread in the container 28 through the through-holes. In this way, by continuing the electrolysis for a certain time, the water in the container 28 can be turned into sterile water rich in hydroxide ions (OH⁻). Furthermore, if hydrogen water is generated in the container 28, and the above-described operation of generating sterile water is performed on the hydrogen water, mixed water of hydrogen water and sterile water can be generated. The mixed water generated in this way is rich in hydrogen and hydroxide ions.

The hydrogen water generated in the container 28 is potable, and the sterile water generated in the container 28 can be used to sterilize a food, such as a vegetable, or various containers for beverages, foods or the like. The sterilization may be performed by immersing an object to be sterilized in the sterile water in the container 28 or by pouring the sterile water in the container 28 over an object to be sterilized. With the hydrogen water and sterile water-generating device 2 in Figure 2(a), a power supply mechanism 29 is provided separately from a main unit part including the container 28 and the electrolysis part 20, the main unit part is placed on the power supply mechanism 29, and the electrolysis can be achieved by the power supply mechanism 29 energizing the electrode (A) 21 and the electrode (B) 22 of the electrolysis part 20. The power supply mechanism may have a battery, such as a dry battery or a storage battery, housed in the main unit part or directly receive electric power from a wall outlet in a house. The hydrogen water and sterile water-generating device according to the present invention can generate hydrogen water and sterile water by electrolysis even at a low voltage from 5 to 15 V, so that a storage battery can be housed in the main unit part, and electric power can be supplied via an USB connector and used by boosting the voltage. In addition, since the electrolysis is achieved by introducing water to only one of the electrodes, the intensity of the current can be reduced compared with the case where water is introduced to both the electrodes, so that the service life of the battery can be extended. Therefore, the hydrogen water and sterile water-generating device is suitable as a portable hydrogen water and sterile water-generating device. In the case of the portable hydrogen water and sterile water-generating device, the thickness of the electrodes is preferably 0.3 to 1 mm, more preferably 0.3 to 0.7 mm, or even more preferably 0.4 to 0.6 mm. In addition, when the voltage is low, the generation of ozone is reduced compared with when the voltage is high, so that the amount of ozone gas discharged can be reduced when generating hydrogen water, and the sterilization effect of the hydroxide ion can be increased when generating sterile water. Furthermore, the hydrogen water and sterile water-generating device 2 can be used as an electric pot if the main unit part includes a water boiling mechanism, such as an electric heater, and the hydrogen water and sterile water-generating device is configured so that a lid can be attached thereto. As the water boiling mechanism, any conventionally known mechanism can be used as required. Figure 2(b) is a diagram showing a hydrogen water and sterile water-generating device 2' that includes a heating plate H provided below the electrolysis part 20 as the water boiling mechanism. When the hydrogen water and sterile water-generating device is used as an electric pot, the hydrogen water and sterile water-generating device may further include a heat insulation mechanism. When the hydrogen water and sterile water-generating device is used as an electric pot, water is put in the container 28 and boiled by the water boiling mechanism, and the above-described operation of generating hydrogen water is then performed after stopping the energization of the water boiling mechanism. In this way, hot water can be turned into hydrogen water. When hot water is used, an ion exchange membrane 25 that is resistant to the temperature of the hot water is used. When the hydrogen water and sterile water-generating device is used as an electric pot, the thickness of the electrodes is preferably 1.5 to 2.5 mm, or more preferably 1.7 to 2.2 mm. Although the hydrogen water or sterile water can be generated at a voltage of 5 to 15 V, the voltage can be further raised to 5 to 24 V for the heating by the heater when the hydrogen water and sterile water-generating device is used as an electric pot.

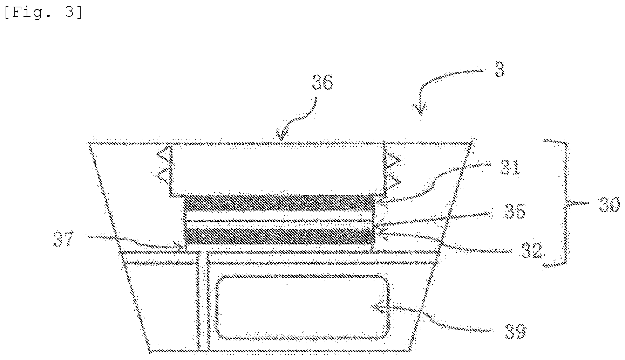

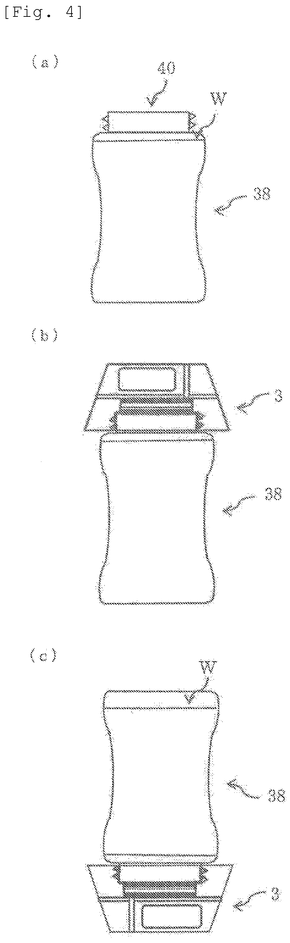

A hydrogen water and sterile water-generating device according to another embodiment of the present invention is characterized in that the hydrogen water and sterile water-generating device comprises an opening part to which a mouth part of a container for containing water is removably attached, an electrolysis part is in communication with the opening part, the water in the container is introduced into the electrolysis part when the container is attached to the opening part, and the water in the container is turned into hydrogen water, sterile water, or mixed water of hydrogen water and sterile water by electrolysis of the introduced water. Figure 3 is a schematic diagram showing an example of the hydrogen water and sterile water-generating device according to this embodiment of the present invention. An electrolysis part 30 of a hydrogen water and sterile water-generating device 3 has an electrode (A) 31, an electrode (B) 32, and an ion exchange membrane 35. The hydrogen water and sterile water-generating device 3 is the same as the hydrogen water and sterile water-generating device 1 shown in Figure 1(a) in the fact that the electrode (A) 31 and the electrode (B) 32 are each a porous electrode plate and the structure of the electrodes, that the ion exchange membrane 35 is a cation exchange membrane, the relationship between the electrodes (A) 31 and (B) 32, the ion exchange membrane 35 and the water introducing channel, that any conventionally known mechanism can be used as the switch mechanism as required, and that an anion exchange membrane can also be used. Figures 4 are schematic diagrams showing the hydrogen water and sterile water-generating device in Figure 3 attached to a container. The hydrogen water and sterile water-generating device 3 includes an opening part 36 to which a mouth part 40 of a container 38 is removably attached, the opening part 36 is in communication with the electrolysis part 30, and water in the container 38 is introduced into the electrolysis part 30 when the container 38 is attached to the opening part 36. In this embodiment, the opening part 36 serves as an introducing channel. As an attachment and detachment structure for the mouth part 40 and the opening part 36, any conventionally known structure can be used, such as a structure in which one of the parts having a male thread formed thereon is screwed into the other part having a female thread formed thereon, or a structure in which one of the parts is fitted into the other part. When water is put in the container 38, the container 38 is attached to the opening part 36, and a voltage is applied between the electrode (A) 31 as a negative electrode (minus electrode) and the electrode (B) 32 as a positive electrode (plus electrode), the water introduced into the electrolysis part 30 through the through-holes of the electrode (A) 31 is electrolyzed. Generated hydrogen ions (H⁺) are attracted by the electrode (A) 31 and turned into hydrogen molecules, which are dissolved in the water in the container 38 through the through-holes of the electrode (A) 31. In this way, by continuing the electrolysis for a certain time, the water in the container 38 can be turned into hydrogen water. Ozone gas (O₃) is discharged to a gas discharge channel 37 through the through-holes of the electrode (B) 32. The gas discharge channel 37 is in communication with the through-holes of the electrode (B) 32, to which the water introducing channel is not connected, at one end thereof and is in communication with the outside of the hydrogen water and sterile water-generating device 3 at the other end thereof. Therefore, the gas generated at the electrode (B) 32 can be discharged to the outside of the hydrogen water and sterile water-generating device 3.

When the container 38 containing water is attached to the opening part 36, and the polarities of the electrode (A) 31 and the electrode (B) 32 are switched between positive and negative by the switch mechanism to set the electrode (A) 31 to be a positive electrode (plus electrode) and set the electrode (B) 32 to be a negative electrode (minus electrode), hydroxide ions (OH⁻) generated by the electrolysis are attracted by the electrode (A) 31 and spread in the container 38 through the through-holes. In this way, by continuing the electrolysis for a certain time, the water in the container 38 can be turned into sterile water rich in hydroxide ions (OH⁻). Furthermore, if hydrogen water is generated in the container 38, and the above-described operation of generating sterile water is performed on the hydrogen water, mixed water of hydrogen water and sterile water can be generated. The mixed water generated in this way is rich in hydrogen and hydroxide ions.

Figures 4 are schematic diagrams showing a way in which the hydrogen water and sterile water-generating device 3 is attached to the container 38. Water is put in the container 38, and the container 38 is placed on a table with the mouth part 40 facing up. Figure 4(a) shows this state. In this state, as shown in Figure 4(b), the hydrogen water and sterile water-generating device 3 is then attached to the container 38 by engaging a threaded part of the mouth part and a threaded part of the opening part with each other. The container 38 with the hydrogen water and sterile water-generating device 3 attached thereto is then turned upside down as shown in Figure 4(c). In this state, the water in the container 38 is introduced into the electrolysis part 30 of the hydrogen water and sterile water-generating device 3 and turned into hydrogen water by electrolysis in the manner described above. The container 38 is then turned upside down again, the hydrogen water and sterile water-generating device 3 is removed, and the hydrogen water in the container 38 is provided for drinking. Sterile water and mixed water of hydrogen water and sterile water can also be generated in the same operation. The hydrogen water and sterile water-generating device 3 can also be used as a portable device having a removable container 38. If a plastic bottle is used as the container 38, water in the plastic bottle can be turned into hydrogen water or sterile water.

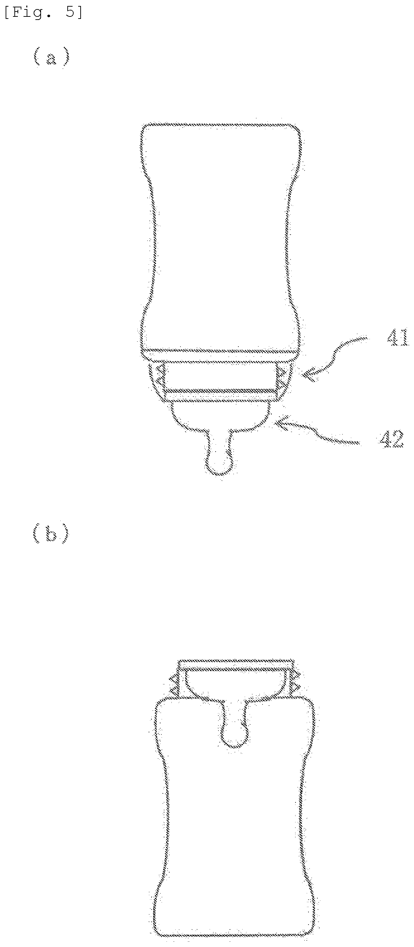

The container 38 is not particularly limited, and can be a baby bottle or a plastic bottle, for example. For example, when the container 38 is a baby bottle, hot water is put in the baby bottle as the container 38. As described above, after the hydrogen water and sterile water-generating device 3 is attached to the baby bottle containing hot water, the baby bottle is turned upside down, and the hot water in the baby bottle is turned into hydrogen water by electrolysis. After that, the baby bottle is turned upside down again, the hydrogen water and sterile water-generating device 3 is removed, and a baby formula is prepared by putting and dissolving a baby formula powder in the hot water in the baby bottle and adjusting the temperature of the hot water. After feeding, the baby bottle is washed with water or hot water, and then water or hot water is put in the baby bottle to generate sterile water in the same operation as described above. The baby bottle can be sterilized by completely filling the baby bottle with the generated sterile water or shaking the baby bottle to clean the inner surface of the baby bottle with the sterile water. Not only the baby bottle but also the nipple of the baby bottle can be sterilized. Figure 5(a) is a diagram showing a nipple 42 being attached to the baby bottle using a cap 41. In a state where the baby bottle contains sterile water, if the baby bottle is turned upside down, the inside of the nipple is immersed in the sterile water, so that the inner surface of the baby bottle can be sterilized. As shown in Figure 5(b), if the baby bottle is filled with sterile water, and the nipple 42 is flipped around and inserted into the mouth of the baby bottle, the outside of the nipple can be immersed in the sterile water, and the outer surface of the baby bottle can be sterilized.

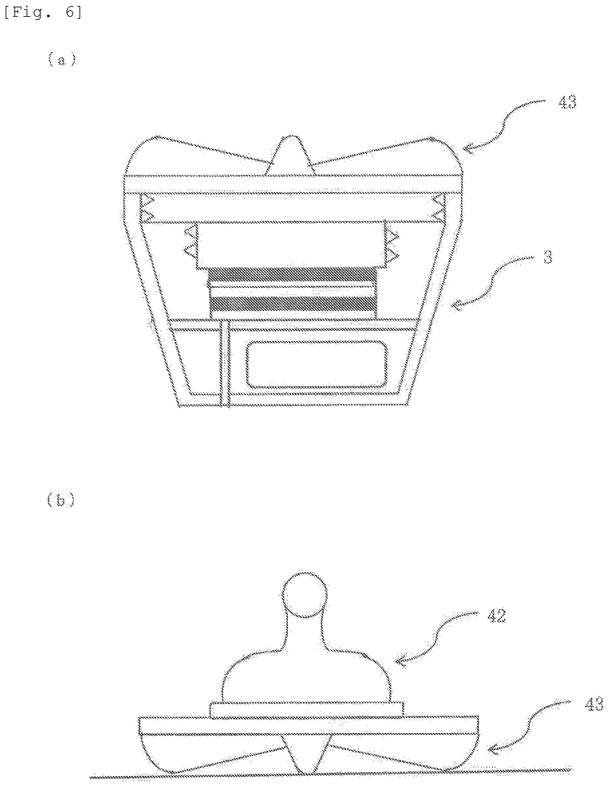

Figure 6(a) is a diagram showing a lid 43 attached to the hydrogen water and sterile water-generating device 3. The lid 43 attached in this way can protect the electrolysis part 30 when the hydrogen water and sterile water-generating device 3 is not in use. Figure 6(b) is a diagram showing an example where the lid 43 is used as a mount on which the nipple is placed when the hydrogen water and sterile water-generating device 3 is used to generate hydrogen water, sterile water or mixed water of hydrogen water and sterile water in the baby bottle. In this way, the nipple can be kept clean. If the hydrogen water and sterile water-generating device 3 is used, after a baby formula is prepared and a feeding is done at a location away from home, the used baby bottle can be sterilized and used again. Therefore, there is no longer a need to carry a plurality of, such as two or three, baby bottles. Furthermore, with a kit containing a set of the hydrogen water and sterile water-generating device 3, a baby bottle, a nipple, and a cap for attaching the nipple to the baby bottle, a baby formula can be appropriately prepared and the used baby bottle can be sterilized at a location away from home or the like. Even when the container 38 is not a baby bottle but another container, such as a plastic bottle, the method of sterilizing the container 38 after sterile water is generated is the same. The power supply mechanism and the thickness of the electrodes are the same as those of the hydrogen water and sterile water-generating device 2.

### Example

Using the hydrogen water and sterile water-generating device 1 shown in Figures 1 whose electrodes have the thickness, the width of the through-holes and the porosity described in the paragraphs [0012] and [0013] described above, hydrogen water, a sterile water, and mixed water of hydrogen water and sterile water were each generated. Tables 1 to 3 show results of operations performed by flowing water processed with a reverse osmotic membrane through the water introducing channel 16. Table 1 shows a result of the operation of generating hydrogen water, Table 2 shows a result of the operation of generating sterile water, and Table 3 shows a result of the operation of generating mixed water. The hydrogen water was generated by flowing 500 mL of water processed with a reverse osmotic membrane through the water introducing channel 16 and electrolyzing the water between the electrode (A) 11 of the hydrogen water and sterile water-generating device 1 as a negative electrode and the electrode (B) 12 as a positive electrode. In this process, the voltage applied to the electrodes was 13 V, and the current was 4 A. Table 1 shows a result of measurements of the water generated by the electrolysis. The polarities of the electrode (A) 11 and the electrode (B) 12 were then switched between positive and negative to set the electrode (A) 11 to be a positive electrode and set the electrode (B) 12 to be a negative electrode, and 500 mL of water processed with a reverse osmotic membrane was flowed through the water introducing channel 16 and electrolyzed. In this step, the voltage applied to the electrodes was 14 V, and the current was 4 A. Table 2 shows a result of measurements of the water generated by the electrolysis. Mixed water was generated by setting the electrode (A) 11 of the sterile water-generating device 1 to be a negative electrode and the electrode (B) 12 to be a positive electrode, flowing 450 mL of water processed with a reverse osmotic membrane through the water introducing channel 16, and then switching the polarities of the electrode (A) 11 and the electrode (B) 12 between positive and negative to set the electrode (A) 11 to be a positive electrode and the electrode (B) 12 to be a negative electrode, and flowing 50 mL of water processed with a reverse osmotic membrane through the water introducing channel 16. In the generated water container located ahead of the introducing channel 16, the hydrogen water and the sterile water were mixed to obtain mixed water containing the hydrogen water and the sterile water in the ratio of 9 to 1. Furthermore, mixed water containing hydrogen water and sterile water in the ratio of 1 to 9 was obtained by setting the electrode (A) 11 of the sterile water-generating device 1 to be a positive electrode and the electrode (B) 12 to be a negative electrode, flowing 450 mL of water processed with a reverse osmotic membrane through the water introducing channel 16, and then switching the polarities of the electrode (A) 11 and the electrode (B) 12 between positive and negative to set the electrode (A) 11 to be a negative electrode and the electrode (B) 12 to be a positive electrode, and flowing 50 mL of water processed with a reverse osmotic membrane through the water introducing channel 16. Table 3 shows a result of measurements of the obtained mixed water. The dissolved hydrogen concentration was measured with a dissolved hydrogen meter (KM2100DH manufactured by Kyoei Electronic Laboratory Co. Ltd.), and the oxidation-reduction potential and pH were measured with a pH/oxidation-reduction potentiometer (HM-31P manufactured by DKK-TOA Corporation). When the polarities of the electrodes were set for generation of hydrogen water, as shown in Table 1, a high-concentration hydrogen water having a dissolved hydrogen concentration higher than 1000 ppb and an oxidation-reduction potential close to -600 was obtained. When the polarities of the electrodes were switched between positive and negative and set for generation of sterile water, as shown in Table 2, the pH decreased and the oxidation-reduction potential increased. The mixed water containing hydrogen water and sterile water in the ratio of 9 to 1 had a lower hydrogen concentration and a higher oxidation-reduction potential than the hydrogen water, which contained no sterile water, but the hydrogen concentration was adequate for use as hydrogen water. The mixed water containing hydrogen water and sterile water in the ratio of 1 to 9 had a higher hydrogen concentration, a lower oxidation-reduction potential and a higher pH than the sterile water, which contained no hydrogen water. Although the obtained sterile water and mixed water (hydrogen water:sterile water = 1:9) did not exhibit a strong acidity, the sterilization effect is considered to be attributed to the hydroxide ion. A similar result is obtained for the hydrogen water and sterile water-generating device 2 shown in Figures 2.

**[Table 1]**

| Generation of Hydrogen Water | | |
|---|---|---|
| | Before Electrolysis | After Electrolysis |
| Water Temperature (°C) | 25.9 | 26.9 |
| Hydrogen Concentration (ppb) | 0 | 1009 |
| ORP(mV) | 337 | -598 |
| pH | 6.12 | 6.64 |

**[Table 2]**

| Generation of Sterile Water | | |
|---|---|---|
| | Before Electrolysis | After Electrolysis |
| Water Temperature (°C) | 25.9 | 27.1 |
| Hydrogen Concentration (ppb) | 0 | 0 |
| ORP(mV) | 337 | 381 |
| pH | 6.12 | 5.69 |

**[Table 3]**

| Generation of Mixed Water | | |
|---|---|---|
| | Hydrogen Water:Sterile Water = 9:1 | Hydrogen Water:Sterile Water = 1:9 |
| Water Temperature (°C) | 27.7 | 28.1 |
| Hydrogen Concentration (ppb) | 826 | 91 |
| ORP(mV) | -556 | 308 |
| pH | 6.14 | 6.10 |

### Industrial Applicability

The hydrogen water and sterile water-generating device according to the present invention can be appropriately used for various applications where hydrogen water is consumed for drinking and various applications where sterile water is used. For example, the hydrogen water and sterile water-generating device according to the present invention can be appropriately used as a portable hydrogen water generator, a hydrogen water producer/server or an electric pot or with a plastic bottle or a baby bottle, for example.

### Explanation of Letters or Numerals

1, 2, 2', 3 hydrogen water and sterile water-generating device
10, 20, 30 electrolysis part
11, 21, 31 electrode (A)
12, 22, 32 electrode (B)
13, 14 through-hole
15, 25, 35 ion exchange membrane
16, 26 water introducing channel
36 opening part (water introducing channel)
17, 27, 37 discharge channel
28, 38 container
29, 39 power supply part
40 mouth part of container
41 cap of baby bottle
42 nipple
43 lid of hydrogen water and sterile water-generating device
H heating plate
W water surface

## Claims

1. A hydrogen water and sterile water-generating device, comprising an electrolysis part that has two electrodes and electrolyzes water, a water introducing channel that introduces water into the electrolysis part, a switch mechanism that switches a polarity of the electrodes between positive and negative, and an ion exchange membrane arranged between the two electrodes, wherein the electrodes are electrodes having a through-hole, the water introducing channel is connected to only one of the electrodes from outside of the electrolysis part, and wherein hydrogen water and sterile water are generated in a same path by a switching of the switch mechanism that switches a polarity of the electrodes between positive and negative during electrolysis.

2. The hydrogen water and sterile water-generating device according to claim 1, wherein the hydrogen water and sterile water-generating device comprises a container for containing water, and has a structure wherein the electrolysis part is in communication with an inside of the container, so that the water in the container is introduced into the electrolysis part, and the water in the container is changed to hydrogen water, sterile water, or mixed water of hydrogen water and sterile water by electrolysis of the introduced water.

3. The hydrogen water and sterile water-generating device according to claim 1, wherein the hydrogen water and sterile water-generating device comprises an opening part to which a mouth part of the container for containing water is removably attached, and has a structure wherein the electrolysis part is in communication with the opening part, so that the water in the container is introduced into the electrolysis part when the container is attached to the opening part, and the water in the container is changed to hydrogen water, sterile water, or mixed water of hydrogen water and sterile water by electrolysis of the introduced water.

4. The hydrogen water and sterile water-generating device according to any one of claims 1 to 3, wherein the ion exchange membrane arranged between the two electrodes are arranged between the two electrodes in contact with the two electrodes.

5. The hydrogen water and sterile water-generating device according to any one of claims 1 to 4, wherein a width of the through-hole of the electrodes is 0.1 to 3 mm.

6. A method of generating mixed water of hydrogen water and sterile water, comprising introducing water into a water electrolysis part from only a side of one of the electrodes, the electrolysis part comprising two electrodes and an ion exchange membrane arranged between the two electrodes, alternately generating hydrogen water and sterile water in a same path by switching a polarity of the electrodes between positive and negative, and mixing the hydrogen water and the sterile water to generate mixed water of hydrogen water and sterile water.

7. The method of generating mixed water of hydrogen water and sterile water according to claim 6, comprising using the hydrogen water and sterile water-generating device according to any one of claims 1 to 5.

8. The hydrogen water and sterile water-generating device according to claim 2, wherein the hydrogen water and sterile water-generating device is an electric pot comprising a water boiling mechanism.

9. The hydrogen water and sterile water-generating device according to claim 3, wherein the container is a baby bottle.

10. A kit of a baby bottle and a hydrogen water and sterile water-generating device, comprising a baby bottle and the hydrogen water and sterile water-generating device according to claim 9.
